# EUROPÄISCHE PATENTSCHRIFT

(11) **EP 1 416 868 B1**
(45) Veröffentlichungstag und Bekanntmachung des Hinweises auf die Patenterteilung: **11.01.2006**
(21) Anmeldenummer: 02772147.1
(22) Anmeldetag: 13.08.2002
(51) Int. Cl.: A61B 17/74

(54) **INTRAMEDULLÄRE OSTEOSYNTHESEEINRICHTUNG ZUR VERSORGUNG VON LATERALEN UND NACH MEDIAL REICHENDEN FEMURFRAKTUREN**
INTRAMEDULAR OSTEOSYNTHESIS DEVICE FOR REPAIRING LATERAL FRACTURES OF THE FEMUR EXTENDING TO THE CENTRE THEREOF
DISPOSITIF D'OSTEOSYNTHESE INTRAMEDULLAIRE POUR REPARER DES FRACTURES LATERALES S'ETENDANT VERS LE CENTRE DU FEMUR

(30) Priorität: 17.08.2001 DE 20113345 U
(43) Veröffentlichungstag der Anmeldung: 12.05.2004
(73) Patentinhaber: Tantum AG, 24534 Neumünster (DE)
(72) Erfinder: JENSEN, Harm-Iven, 24214 Noer (DE)
(74) Vertreter: Wenzel & Kalkoff
(86) Internationale Anmeldenummer: PCT/EP2002/009081
(87) Internationale Veröffentlichungsnummer: WO 2003/015649

(56) Entgegenhaltungen:
- DE-B- 1 071 285
- DE-U- 29 811 670
- FR-A- 2 781 360
- US-A- 4 733 654
- US-B1- 6 261 290

## Beschreibung

Die Erfindung betrifft eine intramedulläre Osteosyntheseeinrichtung zur Versorgung von lateralen und nach Medial reichenden Femurfrakturen, umfassend einen in den Markraum des Femurs einführbaren Marknagel, der in seinem proximalen Endbereich eine erste Querdurchbohrung sowie eine zugeordnete zweite Querdurchbohrung aufweist, ein eine Längsachse aufweisendes Stabelement in Form einer Schenkelhalsschraube, die in der ersten Querdurchbohrung axial verschiebbar geführt und mit ihrem medialen Ende zum Verbinden der Frakturfragmente in den Schenkelhals einführbar ist, und ein eine Längsachse aufweisendes Stabelement in Form eines Schenkelhalsstifts, der die zweite Querdurchbohrung durchgreift, axial verschiebbar und mit seinem medialen Ende in den Schenkelhals einführbar ist, wobei der Schenkelhalsstift die Frakturfragmente derart miteinander verbindet, daß sie relativ zueinander gegen Rotation um die Längsachse der Schenkelhalsschraube gesichert werden.

Die Osteosyntheseeinrichtung wird zur operativen Versorgung von Frakturen im proximalen Teil des Femurs verwendet. Gattungsgemäße Einrichtungen sind zum Beispiel aus DE 198 29 228 C1 (Basis für den Oberbegriff des Anspruchs 1), DE 41 41 152 A1, EP 0 640 318 A1, WO 01 39 679 A1 oder US 5 562 666 bekannt. Der Marknagel wird in das Femur eingebracht. Anschließend wird zunächst in die erste Querdurchbohrung die Schenkelhalsschraube und dann in die zweite Querdurchbohrung der Schenkelhalsstift eingebracht. Mittels der Schenkelhalsschraube werden die Hals-Kopf-Fraktursegmente des Femurs aneinandergefügt und fixiert. Der die Endstellen der Fraktur durchgreifende Schenkelhalsstift bewirkt eine Rotationssicherung, indem er verhindert, daß sich die Frakturfragmente relativ zueinander um die Achse der Schenkelhalsschraube drehen können, wodurch Heilung verzögert würde.

Nach Reposition und bei Knochenheilung findet zumeist eine sogenannte Sinterung statt, die insbesondere durch erhebliche Belastungs- oder Muskelkräfte am Hüftgelenk bewirkt sein kann. Es kann eine Längenvariation des Knochens auftreten. Der Knochen kann sich im Bereich der Fraktur um einen Sinterweg, der einige Millimeter betragen kann, verkürzen. Die Schenkelhalsschraube muß der Verkürzung mit axialer Bewegung nachgeben, da andernfalls die Gefahr besteht, daß die Frakturversorgung instabil wird und sogar ein sogenannter Cut-Out entstehen kann, indem die Schenkelhalsschraube und/oder der Schenkelhalsstift den Femurkopf durchstoßen. Infolgedessen ist grundsätzlich eine feste Verbindung der Schenkelhalsschraube mit dem Marknagel zu vermeiden. Andernfalls entstehen Spannungen und damit Heilungskomplikationen. Um infolge der Längsbewegbarkeit ein Lösen der Schenkelhalsschraube unter dynamischer Belastung zu verhindern, wird die Schenkelhalsschraube gegen Rotation um ihre Achse gesichert (EP 0 257 118 B1). Es entfällt dann jedoch die Rotationssicherung zwischen den Frakturfragmenten.

Demgegenüber liegen der Erfindung die Ziele zugrunde, eine intramedulläre Osteosyntheseeinrichtung zur Versorgung von Femurfrakturen, die die Knochenfragmente relativ zueinander gegen Rotation um die Schenkelhausschraube sichert, durch zusätzliche Sicherung hinsichtlich Stabilisierung der Versorgung zu verbessern. Es soll relative Drehung der Schenkelhalsschraube gegenüber dem Schenkelhals verhindert werden. Das heißt, es sollen ein relatives Auswandern der Schenkelhalsschraube quer zu ihrer Achse und/oder ein Lösen der Schenkelhalsschraube in dem Schenkelhals vermieden werden. Dabei soll sie in ihrer Querdurchbohrung verschiebbar bleiben. Insbesondere bei Knochensinterung soll das Cut-Out-Risiko für die Schenkelhalsschraube sowie für den Schenkelhalsstift elimiert und/oder ein Schmerz verursachendes laterales Herauswandern der Schenkelhalsschraube vermieden werden. Auch sollen operative Maßnahmen vereinfacht sein. Die Einrichtung soll einfach bauen und kostengünstig herstellbar sein.

Die Ziele der Erfindung werden in Verbindung mit den Merkmalen der eingangs genannten Osteosyntheseeinrichtung dadurch erreicht, daß die Einrichtung ein frei von dem Marknagel angeordnetes Verbindungsmittel aufweist, das die Schenkelhalsschraube und den Schenkelhalsstift im Bereich ihrer lateralen Enden miteinander verbindet. Erfindungsgemäß werden die Frakturfragmente zusätzlich gegen Bewegung relativ zueinander gesichert. Die lateralen, caudalen Enden der Schenkelhalsschraube und des Schenkelhalsstiftes werden wenigstens in eine Raumrichtung gegen relative Bewegung oder Verschiebung zueinander gesichert. Infolgedessen können sich die Schenkelhalsschraube und der Schenkelhalsstift nur gemeinsam verschieben, und/oder es werden relative Spreiz-, Scher-, Verkantungs- und/oder Torsionsbewegungen zwischen den beiden Stabelementen blockiert. Es ist eine Verbundanordnung von Schenkelhalsschraube und Schenkelhalsstift erzielt, die die relative Lage der beiden Stabelemente zueinander im Bereich der Fraktur erhält und stabilisiert. Es wird sichergestellt, daß die Fraktursegmente auch dann definiert und zuverlässig mittels der Schenkelhalsschraube in Verbindung mit der Schenkelhalsstift-Rotationssicherung verbunden bleiben, wenn erhebliche Belastungs- oder Muskelkräfte am Hüftgelenk wirken. Die Frakturversorgung wird stabil gehalten, und es werden günstige Voraussetzungen zur Knochensinterung geschaffen. Es wird verhindert, daß sich die Schenkelhalsschraube und auch der Schenkelhalsstift durch Mikrobewegung selbsttätig lösen.Unkontrolliertes Zusammenwachsen der Knochen, Behinderung des Heilungsprozesses und/oder Traumatisierung werden vermieden. Das Cut Out-Risiko wird reduziert.

Eine Ausgestaltung besteht darin, daß das Verbindungsmittel die Schenkelhalsschraube und den Schenkelhalsstift derart miteinander verbindet, daß die Schenkelhalsschraube und der Schenkelhalsstift wenigstens nach Lateral nur gemeinsam in ihren Querdurchbohrungen verschiebbar sind. Dadurch wird insbesondere erreicht, daß der Schenkelhalsstift nicht unkontrolliert nach Lateral auswandern kann, so daß der Durchgriff durch die Endstellen der Fraktur und damit die Rotationssperre der Fraktursegmente relativ zueinander um die Schenkelhalsschraube gesichert bleiben. Dies ist insbesondere bei gegenüber der Schenkelhalsschraube kürzeren Schenkelhalsstiften von Bedeutung, die zur Vermeidung von Cut-Out im oberen Bereich des Femur-Kopfes verwendet werden. Kontrollierte Knochenverkürzung durch Sinterung wird zudem in besonderem Maß dadurch sichergestellt, daß die Schenkelhalsschraube und der Schenkelhalsstift mittels des Verbindungsmittels gemeinsam lateral und medial verschiebbar sind.

In weiterer Ausgestaltung ist das Verbindungsmittel so ausgebildet, daß es wenigstens eines der beiden Stabelemente gegen Drehung um seine Längsachse sichert. Insoweit ist es besonders zweckmäßig, den Schenkelhalsstift gegen Rotation an dem Verbindungsmittel festzusetzen, um seine Position zu stabilisieren. Dies ist besonders vorteilhaft in Verbindung mit einem Schenkelhalsstift, der an seinem medialen Ende eine nach oben gerichtete flache Fläche aufweist, um die Aufnahme von Belastungskräften zu begünstigen. Die abgeflachte Fläche wird in Position gehalten. Gemäß einer Ausführungsform der Erfindung kann die Schenkelhalsschraube an dem Verbindungsmittel gegen Rotation um ihre Längsachse gesichert werden.

Eine Gestaltung der Erfindung besteht auch darin, daß das Verbindungsmittel so ausgebildet wird, daß wenigstens eines der beiden Stabelemente um seine Längsachse drehbar ist. Für eine Reihe von Anwendungsfällen soll die Schenkelhalsschraube drehbar um ihre Längsachse bleiben. Gerade auch für diese Ausführung wird mit der erfindungsgemäßen Verbindung der beiden Stabelemente eine zusätzliche Sicherung der Knochenfragmente relativ zueinander gegen Rotation, Torsion, Scherung und/oder Verkanten erreicht.

Vorteilhaft weist das Verbindungsmittel einen die beiden Stabelemente an ihren lateralen Enden verbindenden plattenartigen Steg auf. Um die Schenkelhalsschraube um ihre Längsachse drehbar zu halten, kann ein Drehlager vorgesehen werden, das zweckmäßig eine an dem Steg ausgebildete Durchgangsbohrung und eine diese durchgreifende, in das laterale Ende der Schenkelhalsschraube eingeschraubte Kopf-Halteschraube umfassen kann. Gemäß einer Ausgestaltung weist das Drehlager längs der Schenkelhalsschraube so viel Spiel auf, daß die Schenkelhalsschraube frei um ihre Längsachse drehbar ist. Eine besondere Gestaltung besteht darin, daß das Drehlager eine kappenartige, an einem Verbindungssteg ausgebildete Lageraufnahme umfaßt, in die die Schenkelhalsschraube im Bereich ihres lateralen Endes derart einfaßt, daß dadurch die lateralen Enden der Schenkelhalsschraube und des Schenkelhalsstifts gemeinsam in in bezug zur Längsachse der Schenkelhalsschraube senkrechter Ebene gehalten werden.

Eine erfindungsgemäße Ausführung besteht auch darin, daß das Verbindungsmittel ein Schwenkgelenkmittel umfaßt, das so vorgesehen ist, daß der Schenkelhalsstift an seinem lateralen Ende um wenigstens eine Schwenkachse schwenkbar an der Schenkelhalsschraube angelenkt ist.

Der CCD-Winkel wird durch die Ausrichtung der Querdurchbohrung der Schenkelhalsschraube und deren Paßführung in der Bohrung bestimmt und festgelegt. Um den Schenkelhalsstift axial verschiebbar zu halten, ist es üblich und zweckmäßig, für den Durchgriff durch die zugehörige Längsbohrung in dieser ausreichend Spiel vorzusehen. Zum Vermeiden oder Beheben eines Verkantens bzw. einer Querstellung des Schenkelhalsstiftes ist es in erfindungsgemäßer Ausgestaltung besonders zweckmäßig und vorteilhaft, daß das Verbindungsmittel die Schenkelhalsschraube und den Schenkelhalsstift derart miteinander verbindet, daß diese im Bereich ihre lateralen Enden in festem Radialabstand in bezug zur Längsachse der Schenkelhalsschraube gehalten sind. Dazu trägt erfindungsgemäß zweckmäßig zusätzlich bei, daß die Schenkelhalsschraube und der Schenkelhalsstift an ihren lateralen Enden gemeinsam in zur Längsachse der Schenkelhalsschraube senkrechter Ebene gehalten werden. In üblicher Anordnung sollen sich die Schenkelhalsschraube und der Schenkelhalsstift zumindest im wesentlichen parallel erstrecken. Abweichungen von der Parallelausrichtung werden mit den genannten Maßnahmen zuverlässig und einfach vermieden und/oder nach dem Einbringen des Schenkelhalsstifts durch Anbringung des Verbindungsmittels beseitigt.

Unteransprüche sind auf die genannten und noch andere zweckmäßige und vorteilhafte Ausgestaltungen der Erfindung gerichtet. Besonders zweckmäßig und vorteilhafte Ausbildungsformen oder -möglichkeiten der Erfindung werden anhand der folgenden Beschreibung der in der schematischen Zeichnung dargestellten Ausführungsbeispiele beschrieben. Es zeigen
- Fig. 1 bis 3: Ansichten einer Ausführungsform einer erfindungsgemäßen Osteosyntheseeinrichtung,
- Fig. 4: eine Ansicht gemäß IV-IV in Fig. 1,
- Fig. 5: in einer der AP-Ebene entsprechenden Ebene eine Ansicht einer erfindungsgemäßen Osteosyntheseeinrichtung, die der Ausführung in Fig. 1 bis 4 entspricht,
- Fig. 6: eine Draufsicht auf die Einrichtung der Fig. 5 gemäß VI-VI und
- Fig. 7: eine Ansicht der Einrichtung der Fig. 5 gemäß VII-VII.

Die in den Figuren dargestellte intramedulläre Osteosyntheseeinrichtung umfaßt einen proximal in den Markraum des Femurs 5 einführbaren Marknagel 1, eine Schenkelhalsschraube 2, einen Schenkelhalsstift 3 und ein diese beiden Stabelemente im Bereich ihrer lateralen bzw. distalen Enden verbindendes Verbindungsmittel 4.

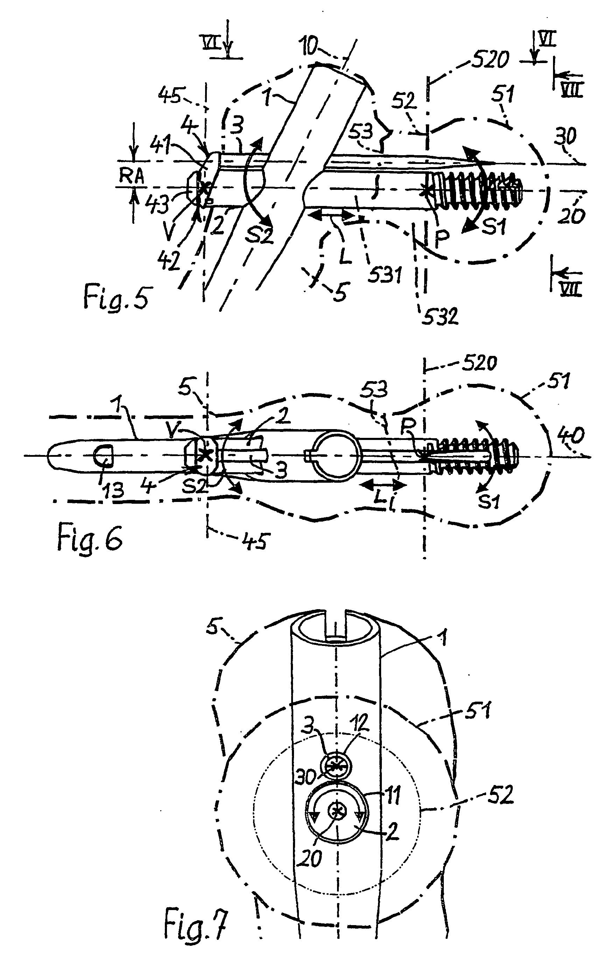

Am Ende des oberen Viertels des Verriegelungsnagels 1 ist eine schräge erste Querdurchbohrung 11 ausgebildet, deren Schräglage dem CCD-Winkel und damit der Femur-Schenkelhalsneigung entspricht. Die Schenkelhalsschraube 2 weist einen zylindrischen Schaftabschnitt auf, mit dem sie passend in der Querdurchbohrung 11 sitzt, wobei sie sowohl um ihre Längsachse 20 drehbar als auch in Axialrichtung L verschiebbar gelagert und geführt ist. An ihrem medialen bzw. proximalen Ende ist die Schenkelhalsschraube 2 mit einem selbstschneidenden Gewinde versehen, mit dem sie zum Verbinden von Hals-Kopf-Frakturfragmenten 531, 532 in den Schenkelhals 52 einführbar ist, um das abgebrochene Hals-Kopf-Fragment 532 zu halten, wie dies aus Fig. 5 bis 7 hervorgeht.

Der Marknagel 1 weist zu der Querdurchbohrung 11 eine parallele zweite Neben-Querdurchbohrung 12 auf, deren Durchmesser geringer ist und durch die der Schenkelhalsstift 3 hindurchgeführt ist. Die Bohrung 12 erstreckt sich zwischen dem proximalen Ende des Marknagels 1 und der Schenkelhalsschraube 2 parallel mit und in geringem Abstand zu der anderen Bohrung 11. Während die erste Querdurchbohrung 11 für die Schenkelhalsschraube 2 eine Führung für die Schenkelhalsschraube 2 bildet, ist die zweite Querdurchbohrung 12 relativ großzügig bemessen.Der größeren Schenkelhalsschraube 2 kommt die Funktion der Winkelstabilität der Schraubenverankerung im Femurkopf 51 zu, und durch axiales Gleiten in ihrer Querdurchbohrung 12 bestimmt sie das Sintern des Femurkopffragments 53. Selbst wenn die Bohrung 11 als genaue Führungspassung für die Schenkelhalsschraube 2 gearbeitet ist, bleibt dennoch minimales Spiel zwischen dem Schaft der Schraube 2 und der Bohrungswand. Einer dadurch möglichen Lockerung der Schraube 2 wird mit dem erfindungsgemäßen Verbindungsmittel 4 begegnet.

Das mediale Ende des nagelartigen Schenkelhalsstifts 3, der einen deutlichen geringeren Durchmesser als die Schenkelhalsschraube 2 aufweist, ist als Flachspitze 31 ausgeformt, um den Stift leichter in den Femurhals 52 eintreiben zu können. Die Spitze 31 ist mit einer abgeflachten Fläche 32 versehen, die der Schenkelhalsschraube 2 abgewandt und nach Proximal gerichtet ist. Diese Abflachung führt zu einer besseren Abstützung des Knochens auf dem Schenkelhalsstift 3, und der Stift 3 wird dadurch an seinem freien Ende gegen Rotation gesichert. Der Schenkelhalsstift 3 ist kürzer als die Schenkelhalsschraube 2 ausgebildet, so daß er weniger weit in den Femurkopf 51 ragt als die Schenkelhalsschraube 2. Statt mit gewindeloser Spitze 31 kann der Schenkelhalsstift 3 auch als Schraube mit selbstschneidendem Gewinde ausgebildet sein.

Der Marknagel 1 weist kreiszylindrischen Querschnitt auf, der im proximalen oberen Viertel relativ groß ist und zum distalen Ende des Nagels 1 hin abnimmt. In dem unteren Bereich des Marknagels 1 ist eine Querdurchbohrung 13 eingebracht. Durch diese wird eine nicht dargestellte Knochenschraube hindurchgesetzt, um den Marknagel 1 nach dem Einführen in das Femur 5 axial und in Drehrichtung zu verriegeln.

Das Verbindungsmittel 4 umfaßt einen plattenartigen Steg 41 mit einem ersten Ende 411 und einem zweiten Ende 412. Der Steg 41 ist ohne Verbindung mit dem Marknagel 1 frei von diesem angeordnet. Im Bereich des ersten Stegendes 411 ist ein Drehlager 42 des Verbindungsmittels 4 vorgesehen, mit dem die Schenkelhalsschraube 2 um ihre Längsachse 20 drehbar an dem Steg 41 gelagert ist. Das zweite Stegende 412 ist starr und infolgedessen auch fest gegen Drehung mit dem lateralen Ende des Schenkelhalsstifts 3 verbunden. So ist das Verbindungsmittel 4 fest mit dem Schenkelhalsstift 3 verbunden. Im Ausführungsbeispiel ist der Steg 41 mit dem Schenkelhalsstift 3 einstückig integral verbunden. Das heißt, daß der Steg 41 und der Schenkelhalsstift 3 durch ein gemeinsames Bauteil gebildet werden. Der Steg 41 kann aber auch auf jede andere geeignete Weise drehfest an dem Schenkelhalsstift 3 befestigt sein. Mit dem Steg 41 werden die Schenkelhalsschraube 2 und der Schenkelhalsstift 3 an ihren lateralen Enden in festem Radialabstand RA in bezug zur Längsachse 20 der Schenkelhalsschraube 2 gehalten. Man erhält zwischen den Stabelement-Längsachsen 20, 30 zumindest im wesentlichen den Parallelabstand RA.

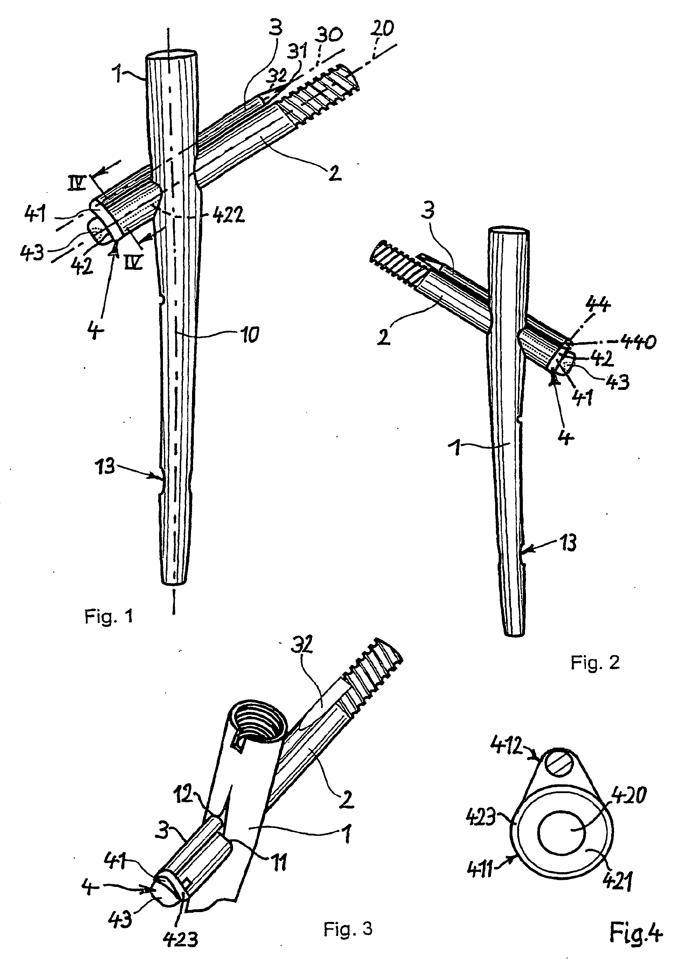

Wie insbesondere aus Fig. 1 und 3 in Verbindung mit Fig. 4 ersichtlich, weist das Drehlager 42 eine an dem ersten Stegende 411 ausgebildete Durchgangsbohrung 420 und eine diese durchgreifende, in das laterale Ende der Schenkelhalsschraube 2 eingeschraubte Kopf-Halteschraube 43 auf. Zum Herstellen der Drehverbindung wird die Kopf-Halteschraube 43 durch die Durchgangsbohrung 420 gesteckt und in eine axiale Gewindebohrung 422 am Ende der Schenkelhalsschraube 2 eingeschraubt. In diesem zusammengebauten Zustand ist der Steg 41 auch mit der Schenkelhalsschraube 2 integral verbunden; das heißt, daß die beiden Bau- und Funktionsteile durch ein gemeinsames Bauteil gebildet werden. Das Drehlager 42 weist eine als Kappe mit Kappenwand 423 geformte, an dem ersten Stegende 411 ausgebildete Lageraufnahme 421 auf. In diese faßt die Schenkelhalsschraube 2 mit ihrem lateralen Ende im Dreh-Paßsitz ein. Die Kopf-Halteschraube 43 ist gegen Anschlag in die Gewindebohrung 422 eingeschraubt und in dieser Verbindungsposition durch Selbsthemmung festgesetzt. Längs der Schenkelhalsschraube 2 besteht zwischen dem Kopf der Halteschraube 42 und der lateralen Endfläche der Schenkelhalsschraube 2 nur soviel Lagerabstand oder -spiel, daß die Schenkelhalsschraube 2 um ihre Längsachse 20 frei drehbar ist, während das Lager 42 so ausgebildet oder eingestellt wird, daß der Steg 41 axial sowohl nach Lateral als auch nach Medial nur gemeinsam mit der Schenkelhalsschraube 2 verschiebbar ist. Anstelle der freien Drehlagerung kann es auch zweckmäßig und vorteilhaft sein, das Drehlager 42 so auszubilden, daß eine die Rotation der Schenkelhalsschraube 2 hemmende Gleit-Reibverbindung hergestellt ist. Ein solches Lager kann mit Einstell- und/oder Klemmitteln ausgestattet werden, um das Maß der Rotations-Gleithemmung bzw. die Reibschlußverbindung nach Bedarf zu bestimmen und einzustellen.

Aus den Fig. 5 bis 7 werden die mit der erfindungsgemäßen Verbindung 4 wahlweise oder in Gesamtheit herstellbaren Bewegungssperren deutlich.

Im Ausführungsbeispiel verbindet das Verbindungsmittels 4 den Schenkelhalsstift 3 in festem parallelem Axialabstand RA mit der Schenkelhalsschraube 2, und die beiden Stabelemente 2, 3 sind relativ zueinander axial unverschiebbar miteinander verbunden. Zu diesem Zweck werden die Längsachse 20 der Schenkelhalsschraube 2 sowie die Längsachse 3 0 des Schenkelhalsstifts 3 durch die zuvor beschriebene Verbindung in diesen beiden Achsen 20, 30 gemeinsamer, von der Achse 20 ausgehender Radialebene 40 gehalten, und die Stabelemente 2, 3 sind an ihren lateralen Achsenden mit dem konstanten Abstand RA und in Axialrichtung L nur gemeinsam verschiebbar miteinander verbunden. Die lateralen Achsenden bleiben stets in gemeinsamer, zur Achse 20 senkrechter Ebene 45 liegen. Infolgedessen werden in alle Raumrichtungen relative Dreh-/Schwenkbewegungen S1 zwischen der Schenkelhalsschraube 2 und dem Schenkelhalsstift 3 um einen gedachten in der Ebene 45 liegenden Verbindungspunkt V gesperrt, über den die lateralen Enden der Stabelemente 2, 3 miteinander verbunden sind. Gleichermaßen werden in alle Raumrichtungen relative Dreh-/Schwenkbewegungen S2 zwischen den Stabelementen 2, 3 um einen gedachten Punkt P im Bereich ihrer medialen Enden gesperrt. Den Punkt P kann man zum Beispiel in einer zur Längsachse 20 der Schenkelhalsschraube 2 senkrecht stehenden Ebene 520 annehmen, die mit einer Halsebene des Femurhalses 52 zusammenfällt. Man erkennt, daß sich die Schenkelhalsschraube 2 in Raumrichtungen S1, S2 nicht relativ zu dem Femurschenkelhals 52 verschwenken bzw. drehen kann, während sie hingegen um ihre Achse 20 drehbar sowie axial verschiebbar und geführt bleibt. Infolgedessen sind erfindungsgemäß zwischen den Stabelementen 2, 3 Scher-, Torsions- und Spreizbewegungen gesperrt. Die aus den Stabelementen 2, 3 und dem Verbindungsmittel 4 gebildete Verschiebeeinheit sichert und stabilisiert die Lage der Frakturfragmente 531, 532 in bezug aufeinander, wobei die Fragmente 531, 532 lediglich in Axialrichtung L durch Mikrobewegungen wandern können, so daß Heilung durch kontrollierte Knochensinterung unterstützt werden kann. Von besonderem Vorteil ist, daß bei der erfindungsgemäßen Einrichtung das zweite Durchgangsloch 12 zum Durchgriff für den Schenkelhalsstift 3 relativ großzügig bemessen werden kann, ohne daß die Rotationssicherung beeinträchtigt wird.

Gemäß einem weiteren Ausführungsbeispiel der Erfindung kann zum Beispiel der Schenkelhalsstift 3 an seinem lateralen Ende auch mit einem Schwenkgelenkmittel 44 um wenigstens eine Schwenkachse 440 schwenkbar an der Schenkelhalsschraube 2 angelenkt werden. Ein solches Schwenkgelenk ist in Fig. 2 fakultativ mit strich-punktierter Linie dargestellt. Bei dieser Ausführungsform sind eine zusätzliche Dreh-/Schwenksicherung durch Sperrung der Bewegungen S2 und durch die nur gemeinsame Axial-Verschiebbarkeit der Stabelemente 2, 3 gewährleistet.

Nach einer Ausführungsform der Erfindung ist es auch möglich, daß das Drehlager 42 beseitigt wird und daß statt dessen das laterale Ende der Schenkelhalsschraube 2 starr oder zumindest in wenigstens einer Umfangsposition mit dem ersten Ende 411 des Steges 41 drehfest verbunden ist, um die Schenkelhalsschraube 2 gegen Drehung um ihre Achse 20 zu sichern.

Eine andere Abänderung des beschriebenen Ausführungsbeispiels kann darin bestehen, daß die Kopf-Halteschraube 43 in eine fixe Position gesetzt wird, in der zwischen ihrem Kopf und der lateralen Endfläche der Schenkelhalsschraube 2 ein bestimmter axialer Abstand eingerichtet wird. Ausgehend von einer Anlageposition der Kappenaufnahme 421 an der lateralen Endfläche der Schenkelhalsschraube 2, ist der Schenkelhalsstift 3 dann mit der Schenkelhalsschraube 2 nur in lateraler Richtung gemeinsam verschiebbar.

Falls gewünscht, kann an Stelle der zuvor beschriebenen drehfesten Verbindung zwischen dem zweiten Stegende 412 auch eine Rotation des Schenkelhalsstiftes 3 um seine Längsachse 30 zugelassen werden.

## Patentansprüche

1. Intramedulläre Osteosyntheseeinrichtung zur Versorgung von lateralen und nach Medial reichenden Femurfrakturen (53), umfassend einen in den Markraum des Femurs (5) einführbaren Marknagel (1), der in seinem proximalen Endbereich eine erste Querdurchbohrung (11) sowie eine zugeordnete zweite Querdurchbohrung (12) aufweist, ein eine Längsachse (20) aufweisendes Stabelement in Form einer Schenkelhalsschraube (2), die in der ersten Querdurchbohrung (11) axial verschiebbar geführt und mit ihrem medialen Ende zum Verbinden der Frakturfragmente (531, 532) in den Schenkelhals (52) einführbar ist, und ein eine Längsachse (30) aufweisendes Stabelement in Form eines Schenkelhalsstifts (3), der die zweite Querdurchbohrung (12) durchgreift, axial verschiebbar und mit seinem medialen Ende in den Schenkelhals (52) einführbar ist, wobei der Schenkelhalsstift (3) die Frakturfragmente (531, 532) derart miteinander verbindet, daß sie relativ zueinander gegen Rotation um die Längsachse (20) der Schenkelhalsschraube (2) gesichert werden, **dadurch gekennzeichnet, daß** die Einrichtung ein frei von dem Marknagel (1) angeordnetes Verbindungsmittel (4) aufweist, das die Schenkelhalsschraube (2) und den Schenkelhalsstift (3) im Bereich ihrer lateralen Enden miteinander verbindet.

2. Einrichtung nach Anspruch 1, **dadurch gekennzeichnet, daß** das Verbindungsmittel (4) die Schenkelhalsschraube (2) und den Schenkelhalsstift (3) derart miteinander verbindet, daß die Schenkelhalsschraube (2) und der Schenkelhalsstift (3) wenigstens nach Lateral nur gemeinsam in ihren Querdurchbohrungen (11, 12) verschiebbar sind.

3. Einrichtung nach Anspruch 2, **dadurch gekennzeichnet, daß** die Schenkelhausschraube (2) und der Schenkelhalsstift (3) auch nach Medial nur gemeinsam in ihren Querdurchbohrungen (11, 12) verschiebbar sind.

4. Einrichtung nach einem der Ansprüche 1 bis 3, **dadurch gekennzeichnet, daß** das Verbindungsmittel (4) die Schenkelhalsschraube (2) und den Schenkelhalsstift (3) derart miteinander verbindet, daß die beiden Stabelemente (2, 3) im Bereich ihrer lateralen Enden in festem Radialabstand (RA) in bezug zur Längsachse (20) der Schenkelhalsschraube (2) gehalten sind.

5. Einrichtung nach einem der Ansprüche 1 bis 4, **dadurch gekennzeichnet, daß** das Verbindungsmittel (4) derart ausgebildet ist, daß die lateralen Enden der Schenkelhalsschraube (2) und des Schenkelhalsstiftes (3) gemeinsam in zur Längsachse (20) der Schenkelhalsschraube (2) senkrechter Ebene (45) gehalten werden.

6. Einrichtung nach einem der Ansprüche 1 bis 5, **dadurch gekennzeichnet, daß** das Verbindungsmittel (4) so ausgebildet ist, daß es wenigstens eines der beiden Stabelemente (2, 3) gegen Drehung um seine Längsachse (20, 3 0) sichert.

7. Einrichtung nach einem der Ansprüche 1 bis 5, **dadurch gekennzeichnet, daß** das Verbindungsmittel (4) so ausgebildet ist, daß wenigstens eines der beiden Stabelemente (2, 3) um seine Längsachse (20, 30) drehbar ist.

8. Einrichtung nach Anspruch 7, **dadurch gekennzeichnet, daß** das Verbindungsmittel (4) ein Drehlager (42) umfaßt, um die Schenkelhalsschraube (2) um ihre Achse (20) drehbar mit dem Schenkelhalsstift (3) zu verbinden.

9. Einrichtung nach Anspruch 8, **dadurch gekennzeichnet, daß** das Drehlager (42) eine an einem Verbindungssteg (41) ausgebildete Durchgangsbohrung (420) und eine diese durchgreifende, in das laterale Ende der Schenkelhalsschraube (2) eingeschraubte Kopf-Halteschraube (43) umfaßt.

10. Einrichtung nach Anspruch 8 oder 9, **dadurch gekennzeichnet, daß** das Drehlager (42) längs der Schenkelhalsschraube (2) so viel Spiel aufweist, daß die Schenkelhausschraube frei um ihre Längsachse (20) drehbar ist.

11. Einrichtung nach Anspruch 8 oder 9, **dadurch gekennzeichnet, daß** das Drehlager so ausgebildet ist, daß eine die Rotation der Schenkelhalsschraube (2) hemmende Gleit-Reibverbindung hergestellt ist.

12. Einrichtung nach einem der Ansprüche 8 bis 11, **dadurch gekennzeichnet, daß** das Drehlager (42) eine kappenartige, an einem Steg (41) ausgebildete Lageraufnahme (421) umfaßt, in die die Schenkelhalsschraube (2) im Bereich ihres lateralen Endes derart einfaßt, daß **dadurch** die lateralen Enden der Schenkelhalsschraube (2) und des Schenkelhalsstifts (3) gemeinsam in zur Längsachse (20) der Schenkelhalsschraube (2) radialer Ebene (40) gehalten werden.

13. Einrichtung nach einem der Ansprüche 1 bis 12, **dadurch gekennzeichnet, daß** das Verbindungsmittel (4) ein Schwenkgelenkmittel (44) umfaßt, das so vorgesehen ist, daß der Schenkelhalsstift (3) an seinem lateralen Ende um wenigstens eine Schwenkachse (440) schwenkbar an der Schenkelhalsschraube (3) angelenkt ist.

14. Einrichtung nach Anspruch 6, **dadurch gekennzeichnet, daß** das Verbindungsmittel (4) so ausgebildet ist, daß wenigstens eines der beiden Stabelemente (2, 3) starr mit dem Verbindungsmittel (4) verbunden ist.

15. Einrichtung nach einem der Ansprüche 1 bis 14, **dadurch gekennzeichnet, daß** das Verbindungsmittel (4) einen Steg (41) mit einem ersten Ende (411) und einem zweiten Ende (412) umfaßt, wobei der Steg (41) an seinem ersten Ende (411) mit dem lateralen Ende der Schenkelhausschraube (2) und an seinem zweiten Ende (412) mit dem lateralen Ende des Schenkelhalsstifts (3) verbunden ist.

16. Einrichtung nach einem der Ansprüche 1 bis 15, **dadurch gekennzeichnet, daß** sich die Schenkelhalsschraube (2) und der Schenkelbalsstift (3) zumindest im wesentlichen parallel erstrecken.

17. Einrichtung nach einem der Ansprüche 1 bis 16, **dadurch gekennzeichnet, daß** der Schenkelhalsstift (3) als nagelartiger Stift ausgebildet ist.

18. Einrichtung nach einem der Ansprüche 1 bis 17, **dadurch gekennzeichnet, daß** der Schenkelhalsstift (3) zwischen dem proximalen Ende des Marknagels (1) und der ersten Querdurchbohrung (11) angeordnet und vorzugsweise auf seiner der Schenkelhalsschraube (2) abgewandten Seite im medialen Endbereich abgeflacht ausgebildet ist.

19. Einrichtung nach einem der Ansprüche 1 bis 18, **dadurch gekennzeichnet, daß** die Schenkelhalsschraube (2) länger als der Schenkelhalsstift (3) ausgebildet ist, so daß die Schenkelhalsschraube (2) nach Medial weiter in den Kopf-Hals-Bereich des Femurs (5) ragt als der Schenkelhalsstift (3).

## Claims

1. Intramedullary osteosynthesis device for the care of femur fractures (53) that are lateral and extending to medial, including a medullary nail (1) which can be introduced into the medullary space of the femur (5) and which in its proximal end region has a first transverse through-bore (11) and an associated second transverse through-bore (12), a rod element having a longitudinal axis (20) and being in the form of a femoral neck screw (2) which is guided axially displaceably in the first transverse through-bore (11) and by its medial end can be introduced into the femoral neck (52) for connecting the fracture fragments (531, 532), and a rod element having a longitudinal axis (30) and being in the form of a femoral neck pin (3) which passes through the second transverse through-bore (12), is axially displaceable and by its medial end can be introduced into the femoral neck (52), wherein the femoral neck pin (3) connects the fracture fragments (531, 532) to each other in such a way that they are secured relative to each other against rotation about the longitudinal axis (20) of the femoral neck screw (2), **characterised in that** the device has a connecting means (4) which is arranged free of the medullary nail (1) and which connects the femoral neck screw (2) and the femoral neck pin (3) to each other in the region of their lateral ends.

2. Device according to claim 1, **characterised in that** the connecting means (4) connects the femoral neck screw (2) and the femoral neck pin (3) to each other in such a way that the femoral neck screw (2) and the femoral neck pin (3) are displaceable in their transverse through-bores (11, 12) only together at least to lateral.

3. Device according to claim 2, **characterised in that** the femoral neck screw (2) and the femoral neck pin (3) are displaceable in their transverse through-bores (11, 12) only together to medial as well.

4. Device according to any of claims 1 to 3, **characterised in that** the connecting means (4) connects the femoral neck screw (2) and the femoral neck pin (3) to each other in such a way that the two rod elements (2, 3) in the region of their lateral ends are held at a fixed radial distance (RA) in relation to the longitudinal axis (20) of the femoral neck screw (2).

5. Device according to any of claims 1 to 4, **characterised in that** the connecting means (4) is designed in such a way that the lateral ends of the femoral neck screw (2) and the femoral neck pin (3) are held together in a plane (45) perpendicular to the longitudinal axis (20) of the femoral neck screw (2).

6. Device according to any of claims 1 to 5, **characterised in that** the connecting means (4) is designed in such a way that it secures at least one of the two rod elements (2, 3) against rotation about its longitudinal axis (20, 30).

7. Device according to any of claims 1 to 5, **characterised in that** the connecting means (4) is designed in such a way that at least one of the two rod elements (2, 3) is rotatable about its longitudinal axis (20, 30).

8. Device according to claim 7, **characterised in that** the connecting means (4) includes a pivot bearing (42) for connecting the femoral neck screw (2) to the femoral neck pin (3) so as to be rotatable about its axis (20).

9. Device according to claim 8, **characterised in that** the pivot bearing (42) includes a through-bore (420) formed on a connecting web (41), and a head-retaining screw (43) passing through this bore and screwed into the lateral end of the femoral neck screw (2).

10. Device according to claim 8 or 9, **characterised in that** the pivot bearing (42) has so much play along the femoral neck screw (2) that the femoral neck screw is freely rotatable about its longitudinal axis (20).

11. Device according to claim 8 or 9, **characterised in that** the pivot bearing is designed in such a way that a sliding friction joint is made, which inhibits rotation of the femoral neck screw (2).

12. Device according to any of claims 8 to 11, **characterised in that** the pivot bearing (42) includes a cap-like bearing receptacle (421) which is formed on a web (41) and in which the femoral neck screw (2) in the region of its lateral end sets in such a way that, as a result, the lateral ends of the femoral neck screw (2) and femoral neck pin (3) are held together in a plane (40) which is radial to the longitudinal axis (20) of the femoral neck screw (2).

13. Device according to any of claims 1 to 12, **characterised in that** the connecting means (4) includes a pivot joint means (44) that is provided in such a way that the femoral neck pin (3) at its lateral end is linked to the femoral neck screw (3) so as to be pivotable about at least one pivot axis (440).

14. Device according to claim 6, **characterised in that** the connecting means (4) is designed in such a way that at least one of the two rod elements (2, 3) is rigidly connected to the connecting means (4).

15. Device according to any of claims 1 to 14, **characterised in that** the connecting means (4) includes a web (41) with a first end (411) and a second end (412), wherein the web (41) is connected at its first end (411) to the lateral end of the femoral neck screw (2) and at its second end (412) to the lateral end of the femoral neck pin (3).

16. Device according to any of claims 1 to 15, **characterised in that** the femoral neck screw (2) and the femoral neck pin (3) extend at least substantially parallel.

17. Device according to any of claims 1 to 16, **characterised in that** the femoral neck pin (3) is designed as a nail-like pin.

18. Device according to any of claims 1 to 17, **characterised in that** the femoral neck pin (3) is arranged between the proximal end of the medullary nail (1) and the first transverse through-bore (11) and is preferably flattened in the medial end region on its side facing away from the femoral neck screw (2).

19. Device according to any of claims 1 to 18, **characterised in that** the femoral neck screw (2) is longer than the femoral neck pin (3), so that the femoral neck screw (2) extends to the medial further into the head-neck region of the femur (5) than the femoral neck pin (3).

## Revendications

1. Dispositif d'ostéosynthèse intramédullaire permettant de traiter des fractures fémorales (53), orientées latéralement et médialement, lequel dispositif comporte une aiguille médullaire (1), qui peut être insérée dans l'espace médullaire du fémur (5) et qui comporte dans sa région d'extrémité proximale un premier perçage traversant transversal (11) et un deuxième perçage traversant transversal associé (12), un élément en forme de barre qui possède un axe longitudinal (20), qui se présente sous la forme d'une vis de col de fémur (2), qui est guidé de façon à pouvoir se déplacer axialement dans le premier perçage traversant transversal (11) et qui peut être inséré par son extrémité médiale dans le col de fémur (52) afin de relier les fragments de fracture (531, 532), et un élément en forme de barre qui possède un axe longitudinal (30) et qui se présente sous la forme d'une tige de col de fémur (3), qui traverse le deuxième perçage traversant transversal (12), qui est déplaçable axialement et qui peut être inséré par son extrémité médiale dans le col de fémur (52), la tige de col de fémur (3) reliant les fragments de fracture (531, 532) de façon à les immobiliser l'un par rapport à l'autre en les empêchant de tourner autour de l'axe longitudinal (20) de la vis de col de fémur (2), **caractérisé en ce que** le dispositif comporte un moyen de liaison (4) qui est disposé librement par rapport à l'aiguille médullaire (1) et qui relie la vis de col de fémur (2) et la tige de col de fémur (3) au niveau de leurs extrémités latèrales.

2. Dispositif sur la revendication 1, **caractérisé en ce que** le moyen de liaison (4) relie la vis de col de fémur (2) et la tige de col de fémur (3) de telle sorte que la vis de col de fémur (2) et la tige de col de fémur (3) ne peuvent se déplacer qu'ensemble, au moins latéralement, dans leur perçage traversant transversal (11, 12).

3. Dispositif selon la revendication 2, **caractérisé en ce que** la vis de col de fémur (2) et la tige de col de fémur (3) ne peuvent se déplacer qu'ensemble également médialement dans leur perçage traversant transversal (11, 12).

4. Dispositif selon l'une des revendications 1 à 3, **caractérisé en ce que** le moyen de liaison (4) relie la vis de col de fémur (2) et la tige de col de fémur (3) de telle sorte que les deux éléments en forme de barre (2, 3) sont maintenus au niveau de leurs extrémités latérales à une distance radiale fixe (RA) par rapport à l'axe longitudinal (20) de la vis de col de fémur (2).

5. Dispositif selon l'une des revendications 1 à 4, **caractérisé en ce que** le moyen de liaison (4) est conformé de telle sorte que les extrémités latérales de la vis de col de fémur (2) et de la tige de col de fémur (3) sont maintenues ensemble dans un plan (45) perpendiculaire à l'axe longitudinal (20) de la vis de col de fémur (2).

6. Dispositif selon l'une des revendications 1 à 5, **caractérisé en ce que** le moyen de liaison (4) est conformé de telle sorte qu'il empêche au moins l'un des deux éléments en forme de barre (2, 3) de tourner autour de son axe longitudinal (20, 30).

7. Dispositif selon l'une des revendications 1 à 5, **caractérisé en ce que** le moyen de liaison (4) est conformé de telle sorte que l'un au moins des deux éléments en forme de barre (2, 3) est apte à tourner autour de son axe longitudinal (20, 30).

8. Dispositif selon la revendication 7, **caractérisé en ce que** le moyen de liaison (4) comporte un palier rotatif (42) permettant de relier la vis de col de fémur (2) à la tige de col de fémur (3) de manière à pouvoir tourner autour de son axe (20).

9. Dispositif selon la revendication 8, **caractérisé en ce que** le palier rotatif (42) comporte un perçage traversant (420) conformé au niveau d'une nervure de liaison (41) et une vis de support de tête (43) traversant ce perçage traversant et vissée dans l'extrémité latérale de la vis de col de fémur (2).

10. Dispositif selon la revendication 8 ou 9, **caractérisé en ce que** le palier rotatif (42) présente le long de la vis de col de fémur (2) un jeu permettant à la vis de col de fémur de pouvoir tourner librement autour de son axe longitudinal (20).

11. Dispositif selon la revendication 8 ou 9, **caractérisé en ce que** le palier rotatif est conformé de façon à réaliser une liaison à frottement par glissement empêchant la rotation de la vis de col de fémur (2).

12. Dispositif selon l'une des revendications 8 à 11, **caractérisé en ce que** le palier rotatif (42) comporte un logement de palier (421) en forme de cuvette qui est conformé au niveau d'une nervure (41) et dans laquelle la vis de col de fémur (2) est montée au niveau de ses extrémités latérales de telle sorte que les extrémités latérales de la vis de col de fémur (2) et de la tige de col de fémur (3) sont maintenues conjointement dans un plan (40) radial par rapport à l'axe longitudinal (20) de la vis de col de fémur (2).

13. Dispositif selon l'une des revendications 1 à 12, **caractérisé en ce que** le moyen de liaison (4) comporte un moyen d'articulation (44) qui est prévu de telle sorte que la tige de col de fémur (3) est articulée sur la vis de col de fémur (3) au niveau de son extrémité latérale de manière à pouvoir pivoter autour d'au moins un axe de pivotement (440).

14. Dispositif selon la revendication 6, **caractérisé en ce que** le moyen de liaison (4) est conformé de telle sorte que au moins l'un des deux éléments en forme de barre (2, 3) est relié rigidement au moyen de liaison (4).

15. Dispositif selon l'une des revendications 1 à 14, **caractérisé en ce que** le moyen de liaison (4) comporte une nervure (41) présentant une première extrémité (411) et une deuxième extrémité (412), la nervure (41) étant reliée à sa première extrémité (411) à l'extrémité latérale de la vis de col de fémur (2) et à sa deuxième extrémité (412) à l'extrémité latérale de la tige de col de fémur (3).

16. Dispositif selon l'une des revendications 1 à 15, **caractérisé en ce que** la vis de col de fémur (2) et la tige de col de fémur (3) s'étendent au moins sensiblement parallèlement.

17. Dispositif selon l'une des revendications 1 à 16, **caractérisé en ce que** la tige de col de fémur (3) est conformée en tige de type aiguille.

18. Dispositif selon l'une des revendications 1 à 17, **caractérisé en ce que** la tige de col de fémur (3) est disposée entre l'extrémité proximale de l'aiguille médullaire (1) et le premier perçage traversant transversal (11) et est avantageusement aplatie dans la région d'extrémité médiale du côté opposé à la vis de col de fémur (2).

19. Dispositif selon l'une des revendications 1 à 18, **caractérisé en ce que** la vis de col de fémur (2) est conformée pour être plus longue que la vis de col de fémur (3) de sorte que la vis de col de fémur (2) s'étend médialement davantage dans la région de col de tête du fémur (5) que la vis de col de fémur (3).
